# EUROPEAN PATENT SPECIFICATION

(11) **EP 1 266 671 B1**
(45) Date of publication and mention of the grant of the patent: **15.09.2004**
(21) Application number: 01850107.2
(22) Date of filing: 15.06.2001
(51) Int. Cl.: A61M 25/01

(54) **Electrically conductive coaxial guide wire**
Elektrisch leitender koaxialer Führungsdraht
Fil-guide coaxial conducteur électrique

(43) Date of publication of application: 18.12.2002
(73) Proprietor: RADI MEDICAL SYSTEMS AB, 754 50 Uppsala (SE)
(72) Inventor: Tenerz, Lars, 756 46 Uppsala (SE)
(74) Representative: Lindgren, Anders

(56) References cited:
- EP-A- 0 673 621
- EP-A- 0 925 803
- US-A- 5 240 437
- US-A- 5 697 380
- US-A- 6 112 598

## Description

The present invention relates generally to guide wire/sensor assemblies, wherein there are at least two electrical leads or conductors required for energizing the sensor and for signal transmission, said leads extending along the length of the guide wire between a proximal end and a distal end. In particular it relates to a coaxial, electrically conductive guide wire provided with an electrical sensor.

### Background of the Invention

For medical purposes many devices and applications have been disclosed and patented, wherein a miniaturized sensor is positioned in the distal region of a guide wire or a catheter. Such sensors can have various purposes, such as the measurement of pressure, temperature, flow, or for the detection of some property in vivo in a living body, e.g. pH, O₂, CO₂ etc.

For most applications it is required that the sensors are energized electrically, and the response is transmitted back to the exterior of the patient as electrical signals. Some applications use optical sensor devices having fiber optic signal transmission, but these fall outside the scope of the present invention.

Thus, some means of signal and energy transmission is required, and most commonly extremely thin electrical leads are provided inside the guide wire, which itself is provided in the form of a tube (of the order of 0.35 mm in outer diameter), oftentimes made of steel. In order to increase the bending strength of the tubular guide wire, a core wire is positioned inside the tube. The mentioned electrical leads are positioned in the space between the inner lumen wall and the core wire.

This design has certain drawbacks:
- it renders the assembly non-rotationally symmetric, since it is virtually impossible to distribute the electrical leads uniformly over the circumference of the core wire inside the space between the inner lumen wall and the core wire. An asymmetric configuration of leads gives rise to different bending properties in different directions of bending;
- there is a risk that the extremely thin leads (although coated with an insulating layer) will become damaged due to friction forces appearing in the lumen, in particular at junctions in the proximal and distal regions, where the core wire changes dimension, and where manipulations during manufacture (e.g. soldering) can cause damage to the coating and possibly cause a short-circuit;
- it is relatively complicated to assemble the device because of the extremely small dimensions of tubes, leads and sensors;
- electrical contacts at the proximal end of the guide wire are made using discrete components, which renders the assembly complicated.

A guide wire disclosed in EP 0 925 803, assigned to the same assignee as the present invention, attempts to solve the symmetry problem by providing electrical leads in the form of concentric layers of conducting material with insulation provide between layers. Although representing an improvement, also this device has certain drawbacks. I.a. there is a risk that the concentric layers may break and cause short-circuit. Another possible problem could be "pinholes" in the very thin insulating layers, representing a potential risk for short-circuiting.

### Summary of the Invention

Thus, in view of the problem with the prior art devices, it is the object of the present invention to provide a guide wire design that overcomes the drawbacks of the prior art sensor/guide wire assembly designs.

This object is achieved with a device as defined in claim 1.

Thereby, a device for measurements, insertable into a living body, is provided which comprises an elongated flexible member having a proximal end and a distal end and a central lumen with an inner wall, a core filling said lumen, an insulating material provided between said core and said inner wall, said core being made of an electrically conductive material and having an essentially constant diameter over its entire length, an electrical sensor attached to the distal end of said elongated flexible member, said electrical sensor being electrically coupled to said core.

By making the guide wire from a coaxial wire, in particular having a central core of essentially constant diameter, the cost of manufacturing becomes lower and manufacturing of the entire device becomes easier.

The manufacturing process can be implemented as a continuous process, wherein long lengths of wire can be made, as opposed to the prior art device, where the wire must be made piece by piece.

Also, the guide wire becomes rotationally symmetric, which is a significant advantage in terms of ease of manipulation of the device by a physician when using the device.

Furthermore, the structure is much less prone to develop "pin holes" that could lead to short circuiting problems.

### Brief Description of the Drawings

The invention will now be described in greater detail with reference to the attached drawings, in which
Fig. 1 is a longitudinal cross-section through a prior art guide wire according to EP 0 925 803;
Fig. 2 is a cross-section through a first embodiment of a coaxial guide wire suitable for use in the invention;
Fig. 3 is a schematic cross-section in the longitudinal direction of a guide wire suitable for use according to the invention;
Fig. 4a shows in greater detail the distal end portion with a sensor mount, and a sensor mounted therein, in a top view;
Fig. 4b shows a cross section in longitudinal direction of the device shown in Fig. 4a;
Fig. 5a shows a cross section at A-A in Fig. 4b;
Fig. 5b shows a cross section at B-B in Fig. 4b;
Fig. 5c shows a cross section at B'-B' in Fig. 4b;
Fig. 6a shows a cross section of a second embodiment of a coaxial guide wire suitable for use in the invention;
Fig. 6b is a longitudinal cross section through a sensor and guide wire assembly according to a second embodiment, namely the distal end portion with a sensor mount, and a sensor mounted therein;
Fig. 7a shows a proximal contact;
Fig. 7b shows a second embodiment of a proximal contact
Fig. 8 illustrates a further embodiment of a sensor and guide wire assembly according to the invention.

### Detailed Description of the Invention

Fig. 1 illustrates a prior art guide wire according to EP 0 925 803 in cross section in the longitudinal direction. It comprises a core wire 2, on which a plurality of concentric layers 4, 6, 8 of conductive material have been provided. Between the conductive layers there are insulating layers 10, 12, 14. In addition to the drawbacks mentioned in the discussion of background of the invention, the process of manufacture is rather complex, either by a complex extrusion process or by a process involving a consecutive deposition of the various layers.

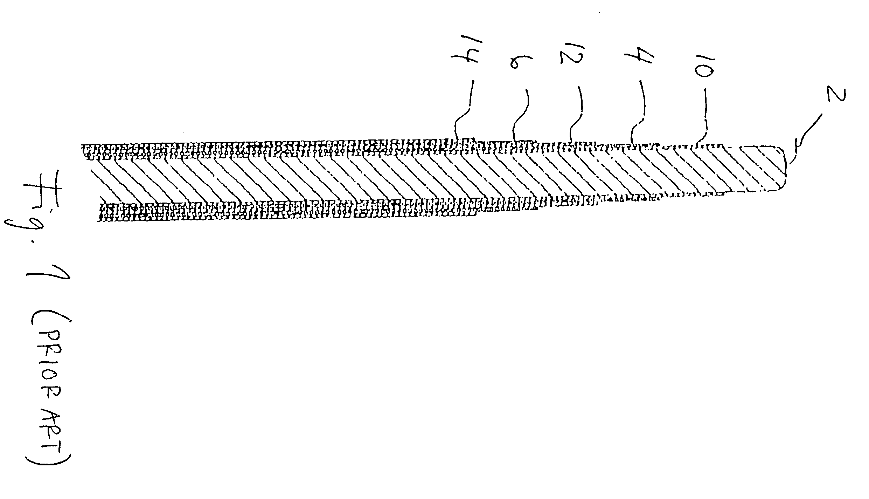

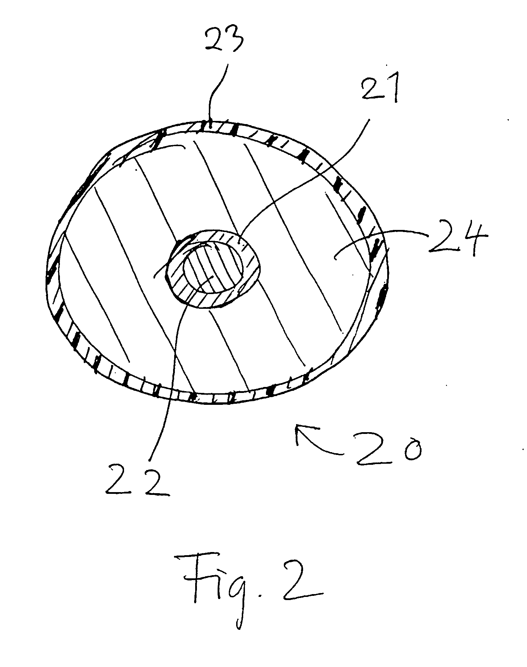

Fig. 2 illustrates the key feature of the present invention. It shows a cross section through a guide wire 20 having a central core 22 of a conductive material, e.g. stainless steel or superelastic metal (e.g. Nitinol®), disposed within the lumen of a thick-walled tube 24 also of a conductive material, e.g. stainless steel or superelastic metal (e.g. Nitinol®), and wherein there is an insulating layer 21 between the core 22 and the tube 24. This structure of the core/insulator/tube can suitably be manufactured by an extrusion process, or by shrinking a tube onto a core. Thus, the core is located inside the tube in a tight fit. The outer surface of the tube 24 is preferably covered by an insulating layer 23, in order to protect the wire from blood and other fluids, which could otherwise cause short circuiting. The insulating material usable with the invention for the outer surface layer 23, can e.g. be selected among a number of polymer materials, Teflon®, poly-imide or Parylene^{TM}, just to mention a few. Also ceramic type materials, e.g. alumina or silicon nitride, in its various possible forms can be used. For the intermediate layer 21, however, the ceramic materials are not suitable, and there polymer materials of the same type as mentioned above are usable.

The diameter of the core over its entire length is constant, and is suitably less than 50% of the outer diameter of the elongated flexible member, preferably less than 25% thereof, except in the distal end, where the outer layer is reduced in order to provided the necessary constructive details, as will be clear from the description below.

The constant diameter of the core is a significant advantage over the prior art structure according to EP 0 925 803, in that it makes possible a continuous manufacturing process. The '803 structure must be made piece by piece. Thus, by using a coaxial wire according to the teachings herein, the manufacturing time will be considerably reduced and simplified, and therefore much more economical.

Other great benefits of this structure for the purpose of making a sensor/guide wire assembly, are that it is rotationally symmetric, the torque can be controlled by controlling the relative dimensions of core and tube, and most importantly, and the mounting of a sensor element on the guide wire becomes very simple, which will be described below in further detail.

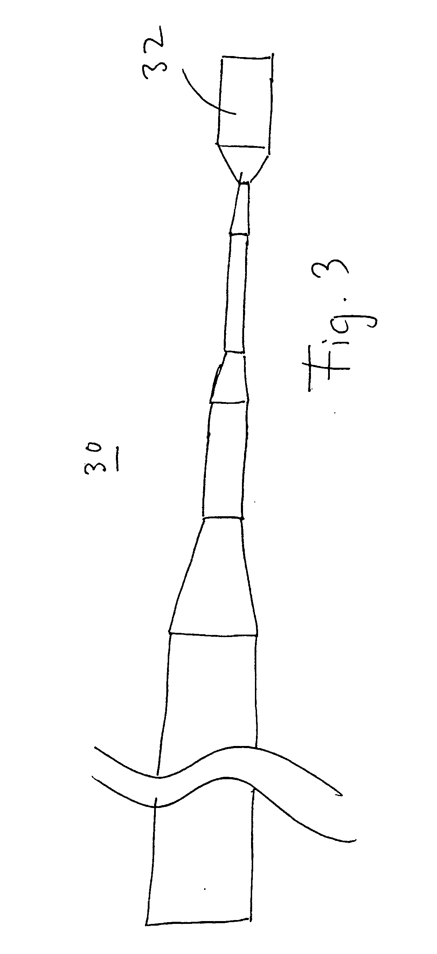

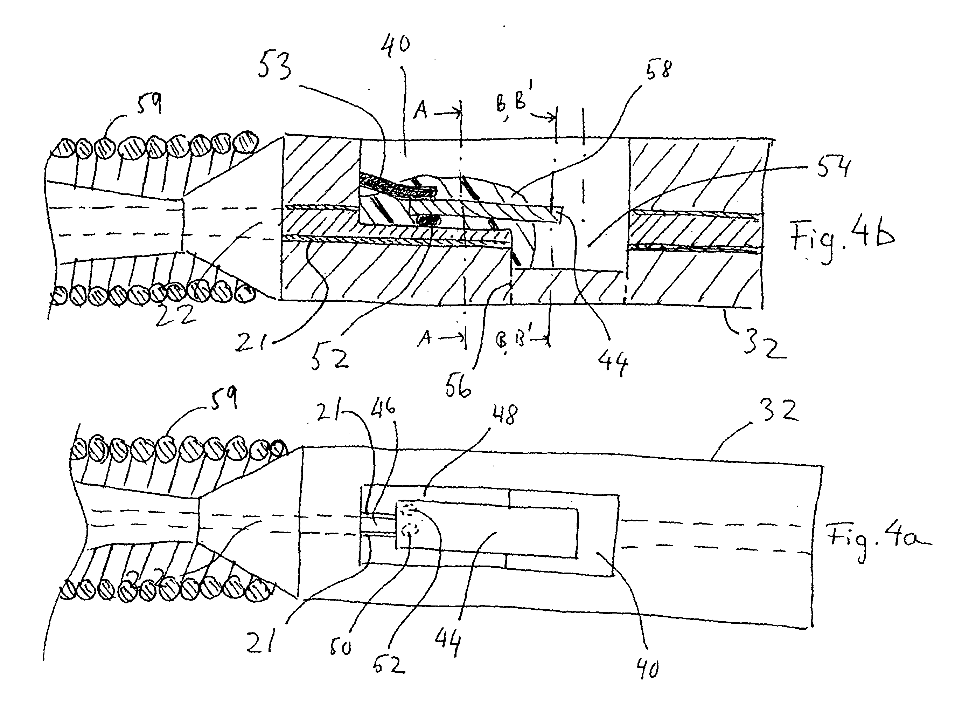

Fig. 3 is a schematic view of an embodiment of an elongated flexible member forming a guide wire, generally designated with reference numeral 30, having the basic construction of Fig. 2, however shown without certain details at the distal end (protective coil, sensor etc). The various segments of the wire are not to shown scale, and the total length of the wire is typically about 1.80 meters, although other lengths are possible, even up to about 3 meters. As can be seen in Fig. 3 the diameter of the elongated flexible member varies, and the in distal end has segments of reduced diameter in order to increase the flexibility of the wire in the distal region. However, the very distal end portion 32 comprises a thicker portion forming a mounting structure for said sensor, and which has a thickness corresponding to the nominal diameter of the wire in its proximal region (e.g. approximately 0.35 mm). This thicker portion comprises a mount (shown in Fig. 4) for a sensor element. This mounting structure comprising a recess, having a bottom surface in a first portion of which the core is exposed so as to form a first contact surface for said sensor, forms an arrangement which is in accordance with the teachings of our US patent No. 6,142,958.

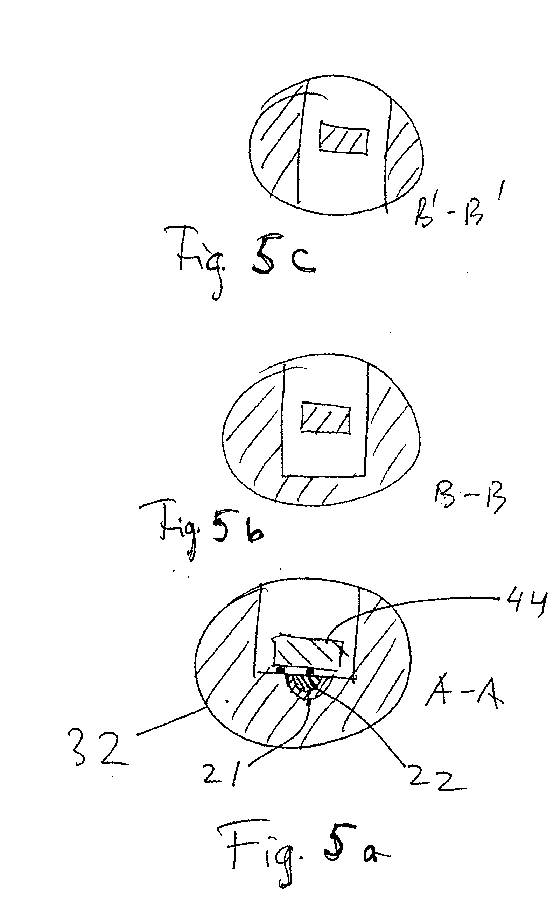

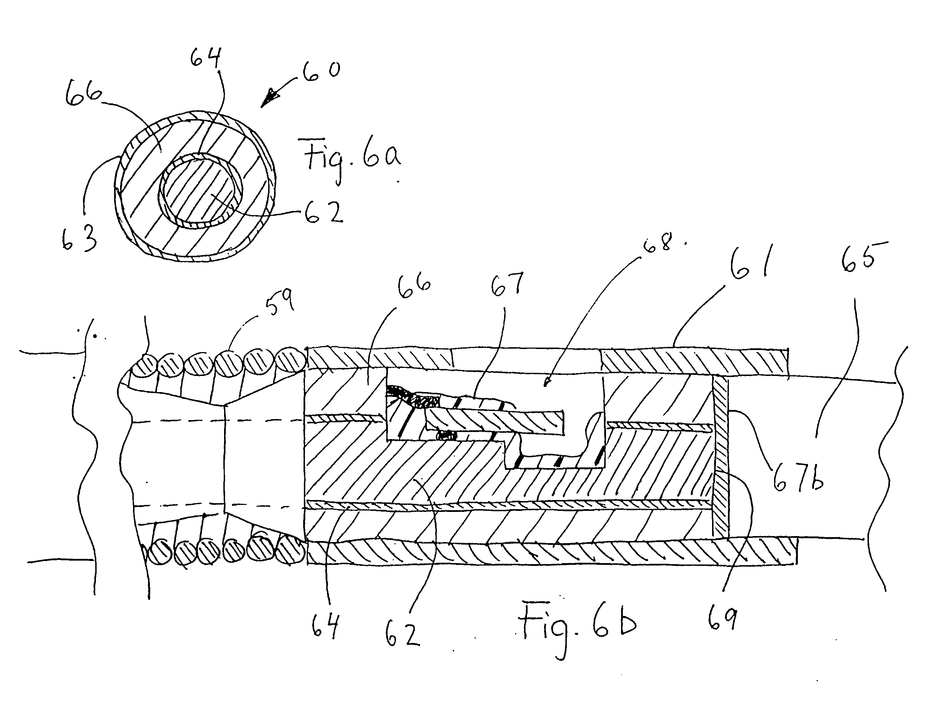

Figs. 4a and 4b illustrates the distal end portion 32 of one embodiment in greater detail, and shows the sensor mount in a top view (Fig. 4a), a side view with parts in cross section (Fig. 4b), and views in cross section at A-A, B-B and B'-B', respectively (Figs. 5a-c).

Thus, the thicker distal sensor mount portion 32 has a recess 40 formed therein, by suitable machining, e.g EDM cutting or laser cutting, which constitutes said sensor mount for a miniaturized sensor 44. Thereby, the bottom surface of the recess will exhibit two regions of conductive material. Namely, the central core 22 (indicated with broken lines in the part not shown in cross section) will be exposed to form a first contact surface 46, separated from the bulk material of the tube by the insulation material 21. The bulk material of the tube will form a second contact surface 48. A sensor chip 44 having two contact terminals 50, 52 (located on the bottom side of the sensor chip, indicated by circles in broken lines), can thus be attached by suitable bonding or soldering to these surfaces, as can be seen in Fig. 4b. In this way, electrical energy can be supplied for energizing purposes in the two leads formed by the core and the tube, respectively. By suitable techniques for modulation of the energizing current, the same leads can be used for signal transmission.

In the shown embodiment the recess 40 has a deeper portion 54 in the region where the sensitive part of the sensor chip 44 is positioned, such that the sensitive part of the chip extends out over the deeper recess part 54, which is seen in Fig. 4b. This is in accordance with the teachings in our patent US- 6,1125,986, and reduces or even eliminates bending artifacts.

The recess in this embodiment reaches through the core 22.

In an alternative design, the deeper recess portion can extend all the way through the thicker portion, as indicated by broken lines 56 in Fig. 4b.

In this embodiment the diameter of the enlarged sensor mount portion 32 has the same diameter as the nominal diameter of the guide wire. Therefore, it is not possible to attach the coil 59 by sliding it over the wire. Instead it must be wound onto the wire at the correct position, i.e. proximally of the sensor mount.

Fig. 5a shows a cross section at A-A in Fig. 4b. Here it can be clearly seen how the sensor chip 44 is attached to the two contact surfaces 46 and 48, respectively, by the contact terminals 50, 52 respectively.

An alternative way if connecting the sensor chip to the outer tube is by bonding an electrical lead 53 thereto. Thereby the contact terminal 52 is dispensed with.

Fig. 5b shows the cross section at B-B in Fig. 4b, which clearly illustrates the "cantilevering" end of the sensor chip.

Fig. 5c is a cross section at B'-B' in Fig. 4b, showing the recess 40 reaching all the way through the entire end portion 32.

In order not to short circuit the core 22 and the outer tube material 32, when the recess is exposed to body fluids, the inner surface of the recess must be covered with some protective material 58. This can be easily achieved by providing silicone or some similar material to cover at least those areas where fluids could cause a short circuit.

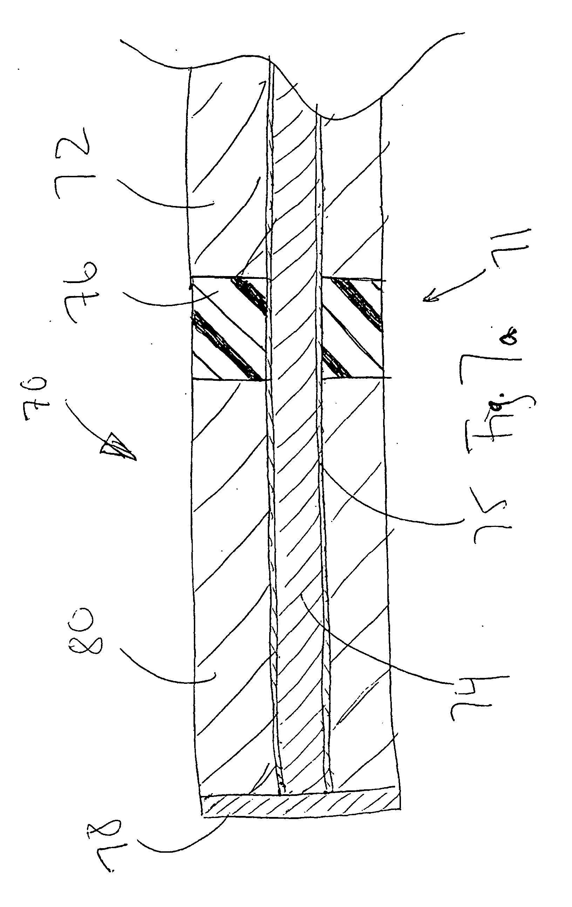

In the embodiment described above with reference to Figs. 4-5, the core has a relatively small diameter compared to the surrounding tube. However, it is also possible to make the coaxial guide wire 60 with a thicker core 62, and a thinner outer tube 66 with an insulating layer 64 provided between the core and the tube, as shown in Fig. 6a. Also here, preferably an insulating layer 63 is applied to the outer surface of the tube 66.

In this case, the recess 68 forming the sensor mount will not reach all the way through the core 62, as can be seen in Fig. 6b. This requires that the entire bottom surface and parts of the walls of the recess 68 be covered with insulating material 67. It also necessitates protection of the distal end surface 69 of the core, in order that the very distal tip 65 that is to be attached thereto will not be in electrical contact with the inner core 62. For the purpose of securing the tip 65, it is convenient to provide a piece of tubing 61 over the enlarged portion forming the sensor mount. This piece of tubing extends slightly beyond the distal end 69, so as to form a cylindrical recess in which the proximal end of the tip 65 can be inserted, and secured by soldering, gluing, or shrinking. Thus, in this case the sensor mount has a diameter that is smaller than the nominal diameter of the wire. Therfore, in this case the coil 59 can be slid over the mount and secured by soldering or gluing, before the piece of tubing 61 is positioned on the mount.

In a further advantageous aspect of the invention, the novel guide wire design provides for a very simple way to construct a proximal male connector, for connecting the guide wire assembly to external equipment.

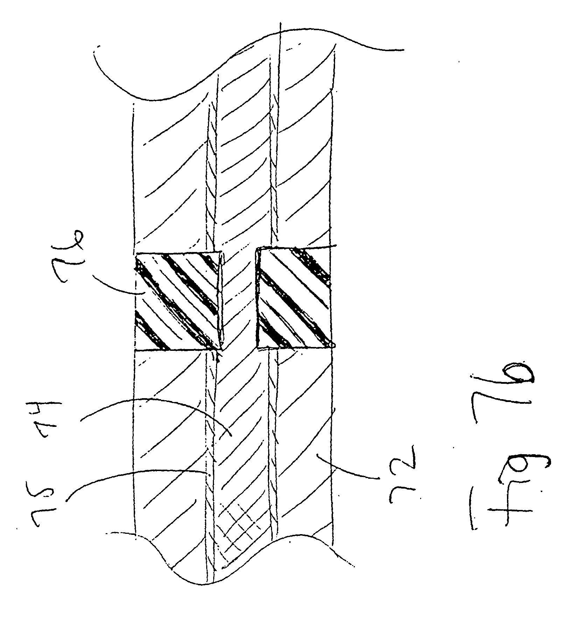

In Fig. 7a the proximal end 70 of a guide wire is shown in cross section. It comprises an outer tube member 72, an inner core 74 with an insulating layer 75 disposed between said core and said tube.

In order to make a connector having two contact elements or surfaces, a circumferential recess 71 is made in the outer tube 72, down to the insulating layer 75 (this embodiment is shown in Fig. 7a), or all the way through the insulating layer 75 down to a depth such that the inner core 74 is exposed (Fig. 7b). It is of course essential that there be no electrical connection between the distal 80 and the proximal 72 part of the outer tube. Then, the recess 72 is filled with an insulating material 76, e.g. some polymer (Teflon® , poly-imide or Parylene or the like) or an inorganic material such as ceramic type materials, e.g. alumina or silicon nitride, in its various possible forms.

Finally, the very end surface of the coaxial wire 80 is "capped" with a layer 78 of conductive material so as to short circuit the core 74 with very end (proximal) portion 80 of the tube, now electrically insulated from the remainder of the tube on the distal side of the recess 71 filled with insulating material 77.

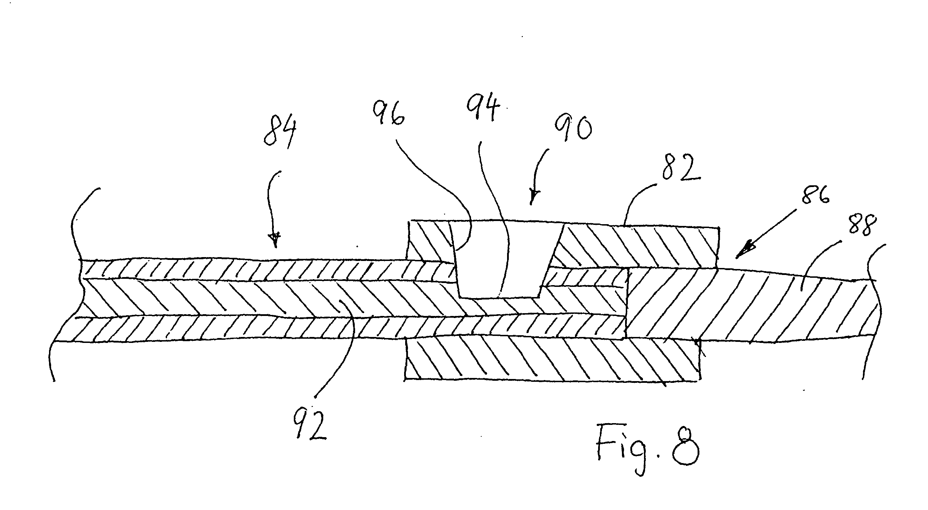

Fig. 8 illustrates an alternative way of providing the sensor mount at the distal end of the wire.

It encompasses providing a tube segment 82 having an inner diameter corresponding to the outer diameter of the coaxial wire 84, and positioning said tube segment 82 over the distal end of the wire 84. Thereby a portion of the tube segment 82 should extend out over the wire 84 so as to form a cylindrical recess 86 at the distal end. This recess will be used for attaching a tip 88 by press fitting and/or soldering.

When the tube 82 is mounted on the wire 84, a recess 90 is provided by suitable techniques, e.g. EDM cutting or laser cutting, down to a depth such that the inner core 92 is exposed. The exposed bottom surface 94 can be used as a first contact surface for a sensor element (not shown). The walls 96 of the recess 90 can be used as a second contact surface for said sensor.

## Claims

1. A guide wire and sensor assembly, insertable into a living body, and having a core of conductive material on which a concentric conductor is provided; wherein
said concentric conductor is an elongated flexible tube (24) made of a conductive material, having a proximal end and a distal end and a central lumen with an inner wall;
said core (22) fills said lumen;
an insulating material (21) is provided between said core (22) and said inner wall;
an electrical sensor (44) is attached to the distal end of said elongated flexible tube,
said electrical sensor (44) being electrically connected (52) to said core (22) and to said tube (24), **characterised in that** said core (22) has an essentially constant diameter over its entire length; and the cross sectional area of said elongated flexible tube member (24) is bigger then that of said insulating material (21).

2. The assembly as claimed in claim 1, wherein the diameter of the elongated flexible tube comprises segments in the distal region having varying diameter, and where the core, except in the distal region, is less than about 50% of the outer diameter of the elongated flexible tube, preferably less than 25% thereof.

3. The assembly as claimed in claim 1 or 2, wherein said core is provided inside said tube in a tight fit.

4. The assembly as claimed in claim 1, 2 or 3, wherein said elongated flexible tube at its distal end comprises a thicker portion forming a mounting structure (32) for said sensor (44), said mounting structure comprising a recess (40), having a bottom surface in a first portion of which the core is exposed so as to form a first contact surface (46) for said sensor (44).

5. The assembly claimed in claim 4, wherein the conductive material of said elongated flexible tube in a second portion of said bottom surface forms a second contact surface (48) for said sensor (44).

6. The assembly as claimed in claim 1,2 or 3, wherein said elongated flexible tube at its distal end comprises a mounting structure for said sensor, said mounting structure comprising a recess, having a bottom surface in a first portion of which the core is exposed so as to form a first contact surface for said sensor, said mounting structure having a diameter that is smaller than the nominal diameter of the wire.

7. The assembly as claimed in claim 6, wherein said elongated flexible tube is made of a conductive material, and wherein the conductive material of said elongated flexible tube in a second portion of said bottom surface forms a second contact surface for said sensor.

8. The assembly as claimed in any preceding claim, wherein said elongated flexible tube is rotationally symmetric.

9. The assembly as claimed in any preceding claim, wherein there is provided a protective material (58; 67) in said recess (40; 68) to prevent short circuit between the core (22) and the tube material (32).

10. The assembly as claimed in any preceding claim, further comprising a connector at the proximal end, comprising a circumferential recess (71) in the outer tube extending at least down to the insulating layer (75), the recess being filled with an insulating material (76) so as to ensure that there is no electrical connection between the proximal part (80) and the distal part (72) of the outer tube, and a proximal end cap (78), short circuiting the core (74) and the proximal part (80) of the tube.

## Patentansprüche

1. Führungsdraht- und Sensoranordnung, die in einen lebenden Körper eingeführt werden kann und eine Ader aus einem leitendem Material besitzt, auf der ein konzentrischer Leiter vorgesehen ist, wobei
es sich bei dem genannten konzentrischen Leiter um eine längliche elastische Hülse (24) handelt, die aus einem leitenden Material besteht und ein proximales und ein distales Ende sowie ein mittiges Lumen mit einer Innenwand besitzt,
die genannte Ader (22) das Lumen ausfüllt,
ein Isoliermaterial (21) zwischen der Ader (22) und der Innenwand vorgesehen ist,
ein elektrischer Sensor (44) an dem distalen Ende der länglichen elastischen Hülse angebracht ist,
der elektrische Sensor (44) mit der Ader (22) und der Hülse (24) elektrisch verbunden ist,
**dadurch gekennzeichnet, dass**
die Ader (22) über ihre gesamte Länge einen im wesentlichen gleichbleibenden Durchmesser hat und die Querschnittsfläche der länglichen elastischen Hülse größer ist als die des Isoliermaterials (21).

2. Anordnung nach Anspruch 1, wobei der Durchmesser der länglichen elastischen Hülse im distalen Bereich Teilstücke mit unterschiedlichem Durchmesser umfasst und die Ader, außer im distalen Bereich, weniger als ungefähr 50%, vorzugsweise weniger als 25%, des Außendurchmessers der länglichen elastischen Hülse im Durchmesser ist.

3. Anordnung nach Anspruch 1 oder 2, wobei die Ader innerhalb der Hülse mit festem Sitz vorgesehen ist.

4. Anordnung nach Anspruch 1, 2, oder 3, wobei die längliche elastische Hülse am distalen Ende einen dickeren Abschnitt umfasst, der eine Befestigungskonstruktion (32) für den Sensor (44) bildet, und die Befestigungskonstruktion eine Vertiefung (40) umfasst, die eine Bodenfläche mit einem ersten Abschnitt besitzt, in dem die Ader freigelegt ist, um so eine erste Kontaktfläche (46) für den Sensor (44) zu bilden.

5. Anordnung nach Anspruch 4, wobei das leitende Material der länglichen elastischen Hülse in einem zweiten Abschnitt der Bodenfläche eine zweite Kontaktfläche (48) für den Sensor (44) bildet.

6. Anordnung nach Anspruch 1, 2 oder 3, wobei die längliche elastische Hülse am distalen Ende eine Befestigungskonstruktion für den Sensor umfasst und die Befestigungskonstruktion eine Vertiefung umfasst, die eine Bodenfläche mit einem ersten Abschnitt besitzt, in dem die Ader freigelegt ist, um so eine erste Kontaktfläche für den Sensor zu bilden, wobei der Durchmesser der Befestigungskonstruktion kleiner ist als der Nenndurchmesser des Drahts.

7. Anordnung nach Anspruch 6, wobei die längliche elastische Hülse aus einem leitenden Material besteht und wobei das leitende Material der länglichen elastischen Hülse in einem zweiten Abschnitt der Bodenfläche eine zweite Kontaktfläche für den Sensor bildet.

8. Anordnung nach einem der vorhergehenden Ansprüche, wobei die längliche elastische Hülse rotationssymmetrisch ist.

9. Anordnung nach einem der vorhergehenden Ansprüche, wobei in der Vertiefung (40, 68) ein Schutzmaterial zur Verhinderung eines Kurzschlusses zwischen der Ader (22) und dem Hülsenmaterial (32) vorgesehen ist.

10. Anordnung nach einem der vorhergehenden Ansprüche, die ferner am proximalen Ende eine Steckvorrichtung mit einer Umfangsvertiefung (71) in der äußeren Hülse umfasst, wobei die Vertiefung mindestens bis zur Isolierschicht (75) hinunter verläuft und mit einem Isoliermaterial (76) gefüllt ist, um so zu gewährleisten, dass zwischen dem proximalen Teil (80) und dem distalen Teil (72) der äußeren Hülse keine elektrische Verbindung besteht, und die eine proximale Endkappe (78) umfasst, die die Ader (74) und den proximalen Teil (80) der Hülse überbrückt.

## Revendications

1. Ensemble capteur et fil guide pouvant être inséré dans un corps vivant, et comportant une âme de matériau conducteur sur laquelle un conducteur concentrique est prévu ; dans lequel
ledit conducteur concentrique est un tube flexible allongé (24) fait d'un matériau conducteur, ayant une extrémité proximale et une extrémité distale et une lumière centrale avec une paroi interne ;
ladite âme (22) remplit ladite lumière;
un matériau isolant (21) est disposé entre ladite âme (22) et ladite paroi interne ;
un capteur électrique (44) est fixé à l'extrémité distale dudit tube flexible allongé,
ledit capteur électrique (44) étant relié électriquement (52) à ladite âme (22) et audit tube (24) **caractérisé en ce que** ladite âme (22) a un diamètre sensiblement constant sur toute sa longueur et la superficie de section transversale dudit tube flexible allongé (24) est plus grande que celle dudit matériau isolant (21).

2. Ensemble selon la revendication 1, dans lequel le diamètre du tube flexible allongé comprend des segments dans la région distale ayant un diamètre variable, et où l'âme, excepté dans la région distale, est inférieure à environ 50 % du diamètre extérieur du tube flexible allongé, étant de préférence inférieur à 25 % de celui-ci.

3. Ensemble selon la revendication 1 ou 2, dans lequel ladite âme est située à l'intérieur dudit tube en un ajustement serré.

4. Ensemble selon la revendication 1, 2 ou 3, dans lequel ledit tube flexible allongé à son extrémité distale comprend une portion plus épaisse formant une structure de montage (32) pour ledit capteur (44), ladite structure de montage comprenant un évidement (40), comportant une surface inférieure dans une première portion de laquelle l'âme est exposée afin de former une première surface de contact (46) pour ledit capteur (44).

5. Ensemble selon la revendication 4, dans lequel le matériau conducteur dudit tube flexible allongé dans une deuxième portion de ladite surface inférieure forme une deuxième surface de contact (48) pour ledit capteur (44).

6. Ensemble selon la revendication 1, 2 ou 3, dans lequel ledit tube flexible allongé à son extrémité distale comprend une structure de montage pour ledit capteur, ladite structure de montage comprenant un évidement, comportant une surface inférieure dans une première portion de laquelle l'âme est exposée afin de former une première surface de contact pour ledit capteur, ladite structure de montage ayant un diamètre qui est inférieur au diamètre nominal du fil.

7. Ensemble selon la revendication 6, dans lequel ledit tube flexible allongé est fait d'un matériau conducteur, et dans lequel le matériau conducteur dudit tube flexible allongé dans une deuxième portion de ladite surface inférieure forme une deuxième surface de contact pour ledit capteur.

8. Ensemble selon l'une quelconque des revendications précédentes, dans lequel ledit tube flexible allongé présente une symétrie de révolution.

9. Ensemble selon l'une quelconque des revendications précédentes, dans lequel est prévu un matériau protecteur (58 ; 67) dans ledit évidement (40 ; 68) pour empêcher un court-circuit entre l'âme (22) et le matériau du tube (32).

10. Ensemble selon l'une quelconque des revendications précédentes, comprenant également un connecteur à l'extrémité proximale, comprenant un évidement circonférentiel (71) dans le tube externe s'étendant au moins jusqu'à la couche isolante (75), l'évidement étant rempli d'un matériau isolant (76) afin de garantir qu'il n'y a pas de connexion électrique entre la partie proximale (80) et la partie distale (72) du tube externe, et un capuchon d'extrémité proximale (78), mettant en court-circuit l'âme (74) et la partie proximale (80) du tube.
